# EUROPEAN PATENT SPECIFICATION

(11) **EP 2 682 459 B1**
(45) Date of publication and mention of the grant of the patent: **20.12.2017**
(21) Application number: 12752828.9
(22) Date of filing: 29.02.2012
(51) Int. Cl.: A61K 35/761, C12N 15/86, A61K 38/00, A61K 31/711, C07K 14/54

(54) **ONCOLYTIC ADENOVIRUS FOR TARGET THERAPY OF HUMAN TUMOR AND USE THEREOF**
ONKOLYTISCHE ADENOVIREN FÜR EINE AUF MENSCHLICHE TUMORE ABZIELENDE THERAPIE UND VERWENDUNG DAVON
ADÉNOVIRUS ONCOLYTIQUE POUR UNE THÉRAPIE CIBLÉE D'UNE TUMEUR HUMAINE, ET UTILISATION ASSOCIÉE

(30) Priority: 02.03.2011 CN 201110050046
(43) Date of publication of application: 08.01.2014
(73) Proprietor: Beijing Bio-Targeting Therapeutics Technology Inc., Beijing 102206 (CN)
(72) Inventor: WANG, Yaohe, Zhengzhou Henan 450001 (CN); JIANG, Guozhong, Zhengzhou Henan 450001 (CN); WANG, Pengju, Zhengzhou Henan 450001 (CN); GAO, Dongling, Zhengzhou Henan 450001 (CN); LEMOINE, Nick, London (GB)
(74) Representative: Hryszkiewicz, Danuta
(86) International application number: PCT/CN2012/071754
(87) International publication number: WO 2012/116636

(56) References cited:
- WO-A1-2007/057368
- WO-A1-2007/068772
- WO-A1-2009/048560
- WO-A2-2004/035799
- WO-A2-2005/086922
- CN-A- 1 258 740
- CN-A- 1 509 764
- CN-A- 1 706 955
- CN-A- 102 174 479
- CN-B- 101 643 750
- HAN HSI WONG ET AL: "Oncolytic Viruses for Cancer Therapy: Overcoming the Obstacles", VIRUSES, vol. 2, no. 1, 11 January 2010 (2010-01-11), pages 78-106, XP055149118, DOI: 10.3390/v2010078
- QIAO J ET AL: "Construction and characterization of a recombinant adenoviral vector expressing human interleukin-12", CANCER GENE THERAPY, APPLETON & LANGE, GB, vol. 6, no. 4, July 1999 (1999-07), pages 373-379, XP002995006, ISSN: 0929-1903, DOI: 10.1038/SJ.CGT.7700061
- ZHANG, WEIPING ET AL: 'THE CONSTRUCTION AND APPLICATION OF ADENOVIRUS VECTOR COEXPRESSING THEHETERODIMEROF HUMAN IL-12' NATL. MED. J. CHINA vol. 78, no. 1, 31 January 1998, pages 33 - 36, XP008170204
- BORTOLANZA, SERGIA ET AL: 'TREATMENT OF PANCREATIC CANCER WITH AN ONCOLYTIC ADENOVIRUS EXPRESSING INTERLEUKIN-12 IN SYRIAN HAMSTERS' MOLECULAR THERAPY vol. 17, no. 4, 17 September 2009, pages 614 - 622, XP055125328
- B. Sangro ET AL: "Phase I Trial of Intratumoral Injection of an Adenovirus Encoding Interleukin-12 for Advanced Digestive Tumors", Journal of Clinical Oncology, vol. 22, no. 8, 15 April 2004 (2004-04-15) , pages 1389-1397, XP055202204, ISSN: 0732-183X, DOI: 10.1200/JCO.2004.04.059
- Sergia Bortolanza ET AL: "Treatment of Pancreatic Cancer With an Oncolytic Adenovirus Expressing Interleukin-12 in Syrian Hamsters", Molecular Therapy, vol. 17, no. 4, 17 February 2009 (2009-02-17), pages 614-622, XP055125328, ISSN: 1525-0016, DOI: 10.1038/mt.2009.9
- HWANG KYUNG-SUN ET AL: "Adenovirus-mediated interleukin-12 gene transfer combined with cytosine deaminase followed by 5-fluorocytosine treatment exerts potent antitumor activity in Renca tumor-bearing mice", BMC CANCER, BIOMED CENTRAL, LONDON, GB, vol. 5, no. 1, 24 May 2005 (2005-05-24), page 51, XP021004813, ISSN: 1471-2407, DOI: 10.1186/1471-2407-5-51

## Description

The invention relates to the field of gene engineering, and more particularly to an oncolytic adenovirus for treating human tumors and the applications thereof.

WO 2007/068772 A1, and Sergia Bortolanza (Sergia Bortolanza et al., Treatment of pancreatic cancer with an oncolytic adenovirus expressing interleukin-12 in Syrian hamsters, Molecular Therapy, Vol. 17, No. 4 (2009), pages 614-622) disclose a conditionally-replicating adenovirus for use in the treatment of pancreatic cancer. The adenovirus includes a fused cDNA sequence of p35 and p40 subunit of human IL12, a promoter operatively linked to the adenoviral E1A gene including at least one hypoxia response element HRE, an E2F responsive promoter operatively linked to the adenoviral E4 gene, and an E1A gene with partial deletion in the CR2 region.

Han Hsi Wong (Han Hsi Wong et al, Oncolytic Viruses for Cancer Therapy: Overcoming the Obstacles, Viruses, Vol. 2, No. 1 (2010), pages 78-106) discloses oncolytic adenoviruses with deletion of E1A, E1B19K, or E1B55K gene.

CN 101 643 750 B discloses a tumor-targeted adenovirus vector Ad-TD-hCCL21. The vector includes deletion of E1A-CR2, E1B19K, E3gp-19K in adenovirus vector Ad5, and human CCL21 gene placed under the control of the E3gp-19K promoter.

WO 2009/048560 A1 discloses a vector for conditionally expressing a protein having the function of interleukin-12. The vector includes a polynucleotide encoding a gene switch.

Qiao J. (Qiao J. et al., Construction and characterization of a recombinant adenoviral vector expressing human interleukin-12 (IL-12), Cancer gene therapy, Appleton & Lance, GB, Vol. 6, No. 4 (1999), pages 373-379) discloses an adenovirus expressing IL-12 under the control of Rous sarcoma virus promoter. The intratumoural delivery of the recombinant adenovirus prolongs the survival time of mice with metastatic colon carcinoma in the liver.

Sangro B. (Sangro B. et al., Phase I trial of intratumoural injection of an adenovirus encoding interleukin-12 for advanced digestive tumours, Journal of Clinical Oncology, Vol. 22, No. 8 (2004), pages 1389-1397) discloses an adenovirus vector expressing IL-12 under the control of cytomegalovirus promoter. The vector is used in the treatment of digestive tumours.

Hwang Kyung-Sun (Hwang Kyung-Sun, et al., Adenovirus-mediated interleukin-12 gene transfer combined with cytosine deaminase followed by 5-flurocytosine treatment exerts potent antitumor activity in Renca tumor-bearing mice, BMC cancer, Biomed central, London, GB, Vol. 5, No. 1 (2005), page 51) discloses adenoviral vectors encoding the p35 and p40 subunits of IL-12 under the control of cytomegalovirus promoter. The adenoviral vectors are used in the treatment of renal cancer.

Adenovirus vector Ad-TD, a kind of tumor-targeted vector created by genetic engineering technology, is a type 5 adenovirus of which genes E1A-CR2, E1B19K, and E3gp-19K are removed, and the promoter sequence of E3gp-19K is retained. The vector has a superior anti-tumor efficacy compared to the first generation of oncolytic adenovirus. Interleukin 12 (IL12) is well-known as a stimulatory factor for Natural Killer Cell and a maturation factor for Cytotoxic T Lymphocytes, and it is a cytokine heterodimer connected by disulfide bonds, and the two subunits are p35 and p40. p35 is produced by T cells, B cells, NK cells, monocytes and other cells; while p40 is mainly produced by activated monocytes and B cells. The sequences of the p35 gene and the p40 gene of human and mouse have been determined, and they have bioactivities as growth factors for NK cells and T cells both *in vivo* and *in vitro.* Further studies have shown that IL12 can effectively inhibit or completely eliminate tumors in mice. However, the half-life of IL12 *in vivo* is very short, so only continuous injection can maintain the therapeutic efficacy, requiring a large amount of IL-12 (1-10 µg/day). Administration of high doses of recombinant IL-12 protein always leads to severe toxicity. Currently, IL12 gene therapy using retroviral vector has been conducted in some laboratories, and its anti-tumor efficacy has been proven. However, direct utilization of IL-12 gene therapy cannot eliminate established tumors and the spontaneous metastases of tumors due to the limited expression of IL12 using the non-replicative vector.

In view of the above-described problems, it is one objective of the invention to provide an adenovirus Ad-TD-hIL12 for targeting treatment of human tumors. The virus can be used as a genetically engineered agent for treating tumors, as the viral vector selectively replicates within tumor cells but not normal cells, and it is safe and highly efficient.

Technical schemes of the invention: The type 5 human adenovirus vector includes an expression cassette with the p35 and p40 subunit genes encoding human IL12 and the virus can selectively replicate in tumor cells and express functional human protein IL12 after infection of tumor cells. These tumors comprise solid tumors, metastatic tumors, and diffusely spreading tumors. The viral vector can selectively amplify in tumor cells, lyse tumor cells, release large amount of tumor-associated antigens, and cooperate with the expressed cytokine human IL12 to effectively induce tumor-specific immunity, which can kill the uninfected tumor cells locally and at remote areas comprising micro-metastatic tumor cells. The highly expressed IL12 in tumor cells can also prevent neovascularization.

To achieve the above objective, in accordance with one embodiment of the invention, there is provided a tumor-targeted adenovirus vector Ad-TD-hIL12. The vector is a type 5 human adenovirus of group C, of which three intrinsic genes E1A-CR2, E1B19K, and E3gp-19Kare removed (Therefore named Ad-Triple Deletion, Ad-TD) and the E3B gene that facilitates the expression of viral genes and enhances the persistence of the viruses *in vivo* is retained. Furthermore, the endogenous promoter of E3gp-19K is retained to drive the expression of the exogenous therapeutic gene - human IL12.

IL12 is a heterodimer comprising a p35 subunit and a p40 subunit, each subunit is encoded by its corresponding gene, and the coding DNA sequences for p35 and p40 subunits of IL12 can be obtained from human cells. The human IL12 sequence in this invention comprises the coding cDNA sequences of IL12 derived from human lymphocytes, *in vitro* synthesized sequences, and recombinant intact or modified coding sequences with anti-tumor efficacy. These sequences comprise but are not limited to point mutation, and internal, 5' and / or 3' end deletion of DNA sequence of human IL12. The homologous DNA sequence or the encoded polypeptide showing anti-tumor efficacy or the sequences inhibiting tumors that are different from the human IL12 sequence in the present invention are also included.

Corresponding amino acid sequences for the p35 and p40 subunit genes are SEQ NO: 1 and SEQ NO: 2 respectively, corresponding nucleotide sequences are SEQ NO: 3 and SEQ NO: 4 respectively.

The oncolytic adenovirus Ad-TD-hIL12 targeted for treating human tumors is used in the applications for preparing drugs for treating renal cancer.

A method for constructing the tumor targeted oncolytic adenovirus Ad-TD-hIL12 for treating human tumors, comprising the following steps:
1) Collecting peripheral blood from healthy donor, isolating and culturing lymphocytes in the presence of phytohemagglutination (PHA), extracting RNA and preparing cDNA by reverse transcription, cloning p35 and p40 subunit cDNA of human IL12 by PCR in the presence of primers including specific enzymatic restriction sites, linking the p35 and p40 subunit gene fragments to yield an intact hIL12 gene fragment, introducing the intact hIL12 gene fragment into a cloning vector to yield pORF-hIL12, digesting the pORF-hIL12 plasmid using two enzymes of Nco I and Nhe I, and blunting the resultant hIL12 gene fragment using T4 DNA polymerase for further cloning;
2) Digesting an adenovirus vector pAd-TD using a blunt end enzyme (SWAI, which is localized between Ad E3 6.9K and the residual region of E3gp-19K), inserting the hIL12 gene into the region by ligation, and identifying the recombinant vectors with correct insertion by PCR; and
3) Transfecting the recombinant vector into 293 cells to produce the infectious viral vector Ad-TD-hIL12.

Advantages of the invention are summarized as follows:
1) The tumor-targeting adenovirus Ad-TD-hIL12 utilizes the endogenous promoter of a viral gene of the virus, three intrinsic adenovirus genes of E1A-CR2, E1B19K, and E3gp-19K are deleted, other genes in the E3 region are retained, and the promoter of the E3gp-19K gene is used to express human IL12 as a therapeutic gene. The vector and therapeutic gene can selectively amplify in tumor cells rather than normal cells by specifically targeting the commonly deregulated Rb gene and anti-apoptosis genes in human tumor cells, thereby ensuring specificity and safety. The replicated viruses can lyse tumor cells and produce high levels of IL12 protein while they replicate within tumor cells, with multiple anti-tumor effects in tumor tissues.
2) The hIL12 expressed by the Ad-TD- hIL12 can prevent neovascularization, and more importantly, it can regulate host immunity and produce a synergistic effect with the replicated Ad-TD-hIL12 in tumor cells, whereby producing specific anti-tumor immunity in organisms, killing metastatic tumor cells from remote places, and preventing recurrence of tumors. Cytological and animal tests show that the virus can selectively kill tumor cells and eradicate tumors established in immune-deficient and immune-competent animals, with an excellent profile of therapeutic efficacy and safety.
3) The tumor-targeted adenovirus Ad-TD-hIL12 in this invention can specifically target tumors and results in superior anti-tumor efficacy. The adenovirus can be used as a targeted, genetically engineered agent for treatment of tumors comprising primary solid tumors, metastatic tumors, and diffusely spreading tumors.
4) The tumor-targeted adenovirus Ad-TD-hIL12 in this invention can not only be used for intratumoral injection, but also be used for intrathoracic injection and intraperitoneal injection, all of which cause no significant side effects.
5) The tumor-targeted adenovirus Ad-TD-hIL12 in this invention is safe and highly efficient, which provides a proof of concept to translate it into clinical applications.

FIG. 1 shows the schematic structures of tumor-targeted adenovirus Ad-TD-hIL12, Ad-TD-gene, and *dl*1520 in accordance with one embodiment of the invention and a structural diagram of a control virus Ad-TD-RFP;
FIG. 2 shows tumor growth curves in Syrian hamster bearing Syrian hamster renal cancer after treatment with low dosages of Ad-TD-hIL12, a control virus and *dl*1520;
FIG. 3 shows the percentage of tumor progression-free animals bearing Syrian hamster-derived tumors after treatments with low dosages of Ad-TD-hIL12, a control virus and *dl*1520;
FIG. 4 shows the tumor eradication rates of tumors in animals bearing subcutaneous Syrian hamster-derived tumors after treatment with low dosages of Ad-TD-hIL12, a control virus and *dl*1520;

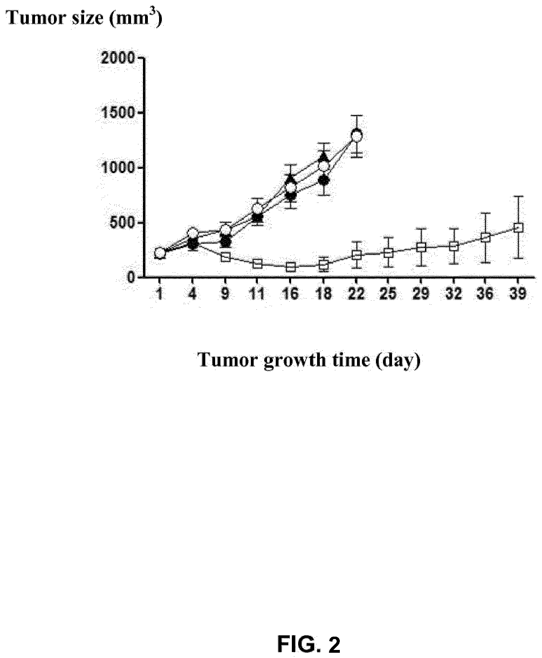

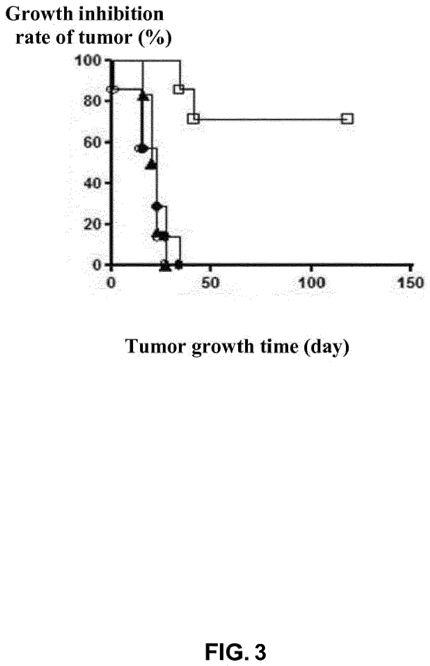

In FIGS. 2 and 3, the following references are used: 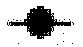 : PBS; 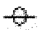 : *dl*1520; 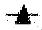 : Ad-TD-RFP; 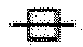 : Ad-TD-hIL12.

For further illustration the invention, experiments detailing an oncolytic adenovirus for treating tumors are described below. It should be noted that the following examples are intended to describe and not to limit the invention.

### Example 1

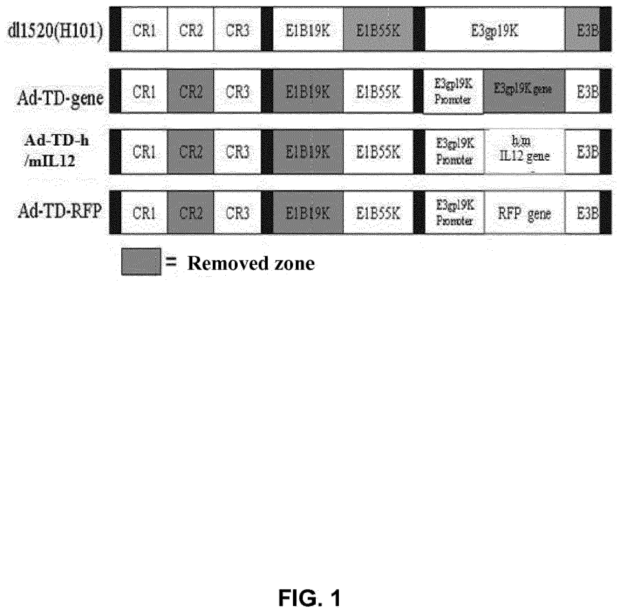

Structure of a tumor-targeted adenovirus Ad-TD-hIL12 (corresponding to human IL12 gene) and Ad-TD-mIL12 (corresponding to mouse IL12 gene), are shown in FIG. 1. The method for constructing the viral vector is described as follows:
(1) First, the DNA fragments at both sides of E1A-CR2 region to be deleted were obtained by PCR, the upstream sequence is named as left arm and the downstream sequence is named as right arm, the left arm and the right arm were ligated with a plasmid pSuperShuttle according to the virus gene sequence by genetic engineering method to construct a shuttle vector of E1A-CR2;
   The adenovirus vector Ad5 and the shuttle vector of E1A-CR2 were transformed into BJ5183 for a homologous recombination at a ratio of 1: 2-10; PCR was performed to identify the positive recombinant bacteria, the recombinant plasmid was extracted and a Ad5R-CR2 viral vector comprising E1A-CR2 depletion is obtained;
(2) The shuttle vector of E1B19K is constructed by using the same method as for constructing the shuttle vector of E1A-CR2, afterwards it was recombined with the viral vector of Ad5R-CR2, and an Ad5R-CR2-E1B19K viral vector with dual depletion of E1A-CR2 and E1B19K was created;
(3) PCR was carried out to amplify the sequences at both sides of the coding region of E3gp-19K gene, the left arm starts from -1087 bp and ends at 0 bp, comprising the promoter of E3gp-19K gene; the right arm starts from 1146 bp downstream the stop codon of E3gp-19K gene, the two arms were connected with enzymatic restriction sites to construct a shuffle vector which was further recombined with the viral vector Ad5ΔR-CR2-E1B19K to obtain an adenovirus vector of pAd-TD with triple depletion of three coding genes E1A-CR2, E1B19K, and E3gp-19K. In addition, pAd-TD has two Swal restriction enzyme sites in the deleted region of E3gp-19K. Thereafter, PacI digested pAd-TD was transfected into 293 cells to produce infectious tumor-targeted adenovirus vector of Ad-TD-gene;
(4) Peripheral blood from healthy donor was collected. The lymphocytes were isolated and cultured in the presence of phytohemagglutination (PHA), the RNA was extracted and reverse-transcripted into cDNA. The primers comprising enzymatic restriction sites were used for cloning p35 and p40 subunit cDNA of human IL12 (or short IL12 or mIL12) by PCR, the p35 and p40 subunit gene fragments were linked by DNA ligase to yield an intact hIL12 gene fragment, which was further inserted into cloning T vector and named as pORF-hIL12 or pORF-shIL12. The plasmids were digested with Nco I and Nhe I to release the coding cDNA fragment of hIL12 or shIL12 or mIL 12 for the next step of cloning;

(5) The deletion region of E3gp-19K of the adenovirus vector of pAd-TD is digested with a blunt end enzyme (SwaI), the hIL12 or shIL12 cDNA coding fragment released from pORF-h/mIL12 vector was inserted into the enzymatic site of the viral vector of pAd-TD in accordance with the genomic sequence, and PCR was carried out to identify the recombinant vector with a correct insertion; and

(6) The recombinant vector with correct insertion was digested with PacI and the viral fragments were transfected into the 293 cells to produce the infectious viral vector Ad-TD-h/mIL12 or Ad-TD-shIL 12.

### Example 2

### Comparison of in vivo anti-tumor efficacy of Ad-TD-hIL12, control virus, and dl1520

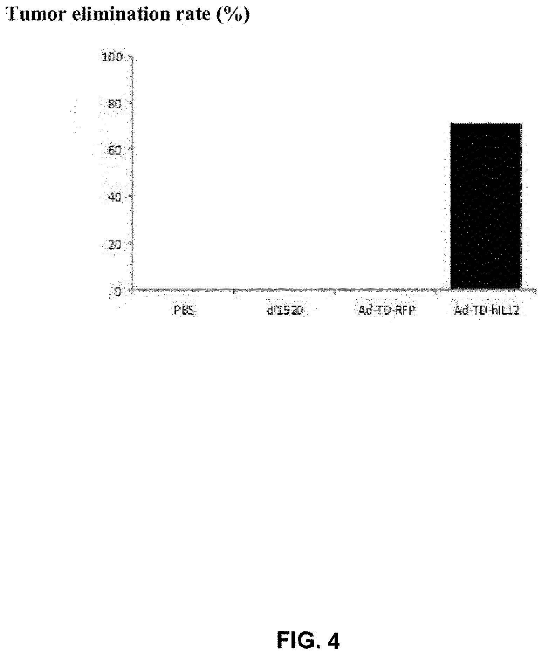

The Syrian hamster renal cancer HAK model was employed. 5×10⁶ of HAK cells were subcutaneously inoculated into the right upper back of 5-6 week old Syrian hamsters (n=7/group). When the tumors approached a relatively larger size (230 mm³), different viruses of lower dosages (1×10E⁹ pt/time, five treatments) were used, and intratumoral injection of PBS, *dl*1520 and Ad-TD-hIL12 was carried out. Regular measurement of the tumor sizes and the tumor elimination rates was performed. The results are shown in FIGS. 2, 3, and 4. FIG. 2 shows that at low dosage, *dl*1520, the control virus Ad-TD-RFP, and PBS had no significant anti-tumor efficacy, while Ad-TD-hIL12 produced a remarkable anti-tumor efficacy. FIG. 3 shows that Ad-TD-hIL12 significantly inhibited the tumor growth in the animals. FIG. 4 shows that the tumor elimination rate in the animals from the Ad-TD-hIL12 treated group is the highest (as high as 71.43%), while the tumor elimination rates in the animals from the *dl*1520 group and the Ad-TD-RFP group were both 0.

## Claims

1. A tumor-targeted adenovirus Ad-TD-hIL12 for use in treating renal cancer, **characterized in that** the type 5 adenovirus comprises the deletion of the three adenovirus genes E1A-CR2, E1B19K, and E3gp-19K and further comprises a fused cDNA sequence of p35 and p40 subunits of human IL12 placed under the control of the E3gp-19K promoter;
wherein
the amino acid sequences of the p35 and p40 subunits are SEQ ID NO: 1 and SEQ ID NO: 2, respectively; and
the nucleotide sequences of the p35 and p40 subunits are SEQ ID NO: 3 and SEQ ID NO: 4, respectively.

## Patentansprüche

1. Auf einen Tumor ausgerichtetes Adenovirus Ad-TD-hIL12 zur Verwendung bei der Behandlung von Nierenkrebs, **dadurch gekennzeichnet, dass** das Adenovirus des Typs 5 die Deletion der drei Adenovirus-Gene E1A-CR2, E1B19K, und E3gp-19K umfasst und ferner eine fusionierte cDNA-Sequenz aus p35- und p40-Untereinheiten von menschlichem IL12 umfasst, die unter die Kontrolle des E3gp-19K-Promotors platziert werden;
wobei
die Aminosäuresequenzen der p35- und p40-Untereinheiten jeweils SEQ-ID NR. 1 und SEQ-ID NR. 2 sind; und
die Nukleotidsequenzen der p35- und p40-Untereinheiten jeweils SEQ-ID NR. 3 und SEQ-ID NR. 4 sind.

## Revendications

1. Adénovirus Ad-TD-hIL12 ciblant une tumeur destiné à être utilisé dans le traitement du cancer du rein, **caractérisé en ce que** l'adénovirus de type 5 comprend la délétion des trois gènes d'adénovirus E1A-CR2, E1B19K et E3gp-19K et comprend en outre une séquence d'ADNc fusionnée des sous-unités p35 et p40 de l'IL12 humaine placée sous le contrôle du promoteur E3gp-19K ;
dans lequel
les séquences d'acides aminés des sous-unités p35 et p40 sont respectivement les SEQ ID NO : 1 et SEQ ID NO : 2 ; et
les séquences nucléotidiques des sous-unités p35 et p40 sont respectivement les SEQ ID NO : 3 et SEQ ID NO : 4.


### SEQUENCE LISTING

<110> Beijing Bio-Targeting Therapeutics Technology Inc.
<120> Oncolytic adenovirus for target therapy of human tumor and use thereof
<130> PCT/CN2012/071754
<160> 4
<170> PatentIn version 3.4
<210> 1
   <211> 198
   <212> PRT
   <213> Homo sapiens
<400> 1
   Ala Arg Asn Leu Pro Val Ala Thr Pro Asp Pro Gly Met Phe Pro Cys
   1               5                   10                  15
   Leu His His Ser Gln Asn Leu Leu Arg Ala Val Ser Asn Met Leu Gln
               20                  25                  30
   Lys Ala Arg Gln Thr Leu Glu Phe Tyr Pro Cys Thr Ser Glu Glu Ile
           35                  40                  45
   Asp His Glu Asp Ile Thr Lys Asp Lys Thr Ser Thr Val Glu Ala Cys
       50                  55                  60
   Leu Pro Leu Glu Leu Thr Lys Asn Glu Ser Cys Leu Asn Ser Arg Glu
   65                  70                  75                  80
   Thr Ser Phe Ile Thr Asn Gly Ser Cys Leu Ala Ser Arg Lys Thr Ser
                   85                  90                  95
   Phe Met Met Ala Leu Cys Leu Ser Ser Ile Tyr Glu Asp Leu Lys Met
               100                 105                 110
   Tyr Gln Val Glu Phe Lys Thr Met Asn Ala Lys Leu Leu Met Asp Pro
           115                 120                 125
   Lys Arg Gln Ile Phe Leu Asp Gln Asn Met Leu Ala Val Ile Asp Glu
       130                 135                 140
   Leu Met Gln Ala Leu Asn Phe Asn Ser Glu Thr Val Pro Gln Lys Ser
   145                 150                 155                 160
   Ser Leu Glu Glu Pro Asp Phe Tyr Lys Thr Lys Ile Lys Leu Cys Ile
                   165                 170                 175
   Leu Leu His Ala Phe Arg Ile Arg Ala Val Thr Ile Asp Arg Val Met
               180                 185                 190
   Ser Tyr Leu Asn Ala Ser
           195
<210> 2
   <211> 328
   <212> PRT
   <213> Homo sapiens
<400> 2
   Met Gly His Gln Gln Leu Val Ile Ser Trp Phe Ser Leu Val Phe Leu
   1 5 10 15
   Ala Ser Pro Leu Val Ala Ile Trp Glu Leu Lys Lys Asp Val Tyr Val
   20 25 30
   Val Glu Leu Asp Trp Tyr Pro Asp Ala Pro Gly Glu Met Val Val Leu
   35 40 45
   Thr Cys Asp Thr Pro Glu Glu Asp Gly Ile Thr Trp Thr Leu Asp Gln
   50 55 60
   Ser Ser Glu Val Leu Gly Ser Gly Lys Thr Leu Thr Ile Gln Val Lys
   65 70 75 80
   Glu Phe Gly Asp Ala Gly Gln Tyr Thr Cys His Lys Gly Gly Glu Val
   85 90 95
   Leu Ser His Ser Leu Leu Leu Leu His Lys Lys Glu Asp Gly Ile Trp
   100 105 110
   Ser Thr Asp Ile Leu Lys Asp Gln Lys Glu Pro Lys Asn Lys Thr Phe
   115 120 125
   Leu Arg Cys Glu Ala Lys Asn Tyr Ser Gly Arg Phe Thr Cys Trp Trp
   130 135 140
   Leu Thr Thr Ile Ser Thr Asp Leu Thr Phe Ser Val Lys Ser Ser Arg
   145 150 155 160
   Gly Ser Ser Asp Pro Gln Gly Val Thr Cys Gly Ala Ala Thr Leu Ser
                   165                 170                 175
   Ala Glu Arg Val Arg Gly Asp Asn Lys Glu Tyr Glu Tyr Ser Val Glu
               180                 185                 190
   Cys Gln Glu Asp Ser Ala Cys Pro Ala Ala Glu Glu Ser Leu Pro Ile
           195                 200                 205
   Glu Val Met Val Asp Ala Val His Lys Leu Lys Tyr Glu Asn Tyr Thr
       210                 215                 220
   Ser Ser Phe Phe Ile Arg Asp Ile Ile Lys Pro Asp Pro Pro Lys Asn
   225                 230                 235                 240
   Leu Gln Leu Lys Pro Leu Lys Asn Ser Arg Gln Val Glu Val Ser Trp
                   245                 250                 255
   Glu Tyr Pro Asp Thr Trp Ser Thr Pro His Ser Tyr Phe Ser Leu Thr
               260                 265                 270
   Phe Cys Val Gln Val Gln Gly Lys Ser Lys Arg Glu Lys Lys Asp Arg
           275                 280                 285
   Val Phe Thr Asp Lys Thr Ser Ala Thr Val Ile Cys Arg Lys Asn Ala
       290                 295                 300
   Ser Ile Ser Val Arg Ala Gln Asp Arg Tyr Tyr Ser Ser Ser Trp Ser
   305                 310                 315                 320
   Glu Trp Ala Ser Val Pro Cys Ser
                   325
<210> 3
   <211> 597
   <212> DNA
   <213> Homo sapiens
<400> 3
   gccagaaacc tccccgtggc cactccagac ccaggaatgt tcccatgcct tcaccactcc      60
   caaaacctgc tgagggccgt cagcaacatg ctccagaagg ccagacaaac tctagaattt     120
   tacccttgca cttctgaaga gattgatcat gaagatatca caaaagataa aaccagcaca     180
   gtggaggcct gtttaccatt ggaattaacc aagaatgaga gttgcctaaa ttccagagag    240
   acctctttca taactaatgg gagttgcctg gcctccagaa agacctcttt tatgatggcc    300
   ctgtgcctta gtagtattta tgaagacttg aagatgtacc aggtggagtt caagaccatg    360
   aatgcaaagc tgctgatgga tcctaagagg cagatctttc tagatcaaaa catgctggca    420
   gttattgatg agctgatgca ggccctgaat ttcaacagtg agactgtgcc acaaaaatcc    480
   tcccttgaag aaccggattt ttataaaact aaaatcaagc tctgcatact tcttcatgct    540
   ttcagaattc gggcagtgac tattgataga gtgatgagct atctgaatgc ttcctaa       597
<210> 4
   <211> 997
   <212> DNA
   <213> Homo sapiens
<400> 4
   atgggtcacc agcagttggt catctcttgg ttttccctgg tttttctggc atctcccctc     60
   gtggccatat gggaactgaa gaaagatgtt tatgtcgtag aattggattg gtatccggat    120
   gcccctggag aaatggtggt cctcacctgt gacacccctg aagaagatgg tatcacctgg    180
   accttggacc agagcagtga ggtcttaggc tctggcaaaa ccctgaccat ccaagtcaaa    240
   gagtttggag atgctggcca gtacacctgt cacaaaggag gcgaggttct aagccattcg    300
   ctcctgctgc ttcacaaaaa ggaagatgga atttggtcca ctgatatttt aaaggaccag    360
   aaagaaccca aaaataagac ctttctaaga tgcgaggcca agaattattc tggacgtttc    420
   acctgctggt ggctgacgac aatcagtact gatttgacat tcagtgtcaa aagcagcaga    480
   ggctcttctg acccccaagg ggtgacgtgc ggagctgcta cactctctgc agagagagtc    540
   agaggggaca acaaggagta tgagtactca gtggagtgcc aggaggacag tgcctgccca    600
   gctgctgagg agagtctgcc cattgaggtc atggtggatg ccgttcacaa gctcaagtat    660
   gaaaactaca ccagcagctt cttcatcagg gacatcatca aacctgaccc acccaagaac    720
   ttgcagctga agccattaaa gaattctcgg caggtggagg tcagctggga gtaccctgac    780
   acctggagta ctccacattc ctacttctcc ctgacattct gcgttcaggt ccagggcaag    840
   agcaagagag aaaagaaaga tagagtcttc acggacaaga cctcagccac ggtcatctgc    900
   cgcaaaaatg ccagcattag cgtgcgggcc caggaccgct actatagctc atcttggagc    960
   gaatgggcat ctgtgccctg cagtgttcct ggagtag                             997